# EUROPEAN PATENT SPECIFICATION

(11) **EP 3 728 177 B1**
(45) Date of publication and mention of the grant of the patent: **20.04.2022**
(21) Application number: 18840071.7
(22) Date of filing: 19.12.2018
(51) Int. Cl.: C07C 51/31

(54) **METHODS OF MAKING POLYFUNCTIONAL POLYFLUORINATED COMPOUNDS**
VERFAHREN ZUR HERSTELLUNG POLYFUNKTIONELLER POLYFLUORIERTER VERBINDUNGEN
PROCÉDÉS DE FABRICATION DE COMPOSÉS POLYFLUORÉS POLYFONCTIONNELS

(30) Priority: 21.12.2017 US 201762609064 P
(43) Date of publication of application: 28.10.2020
(73) Proprietor: 3M Innovative Properties Company, Saint Paul, Minnesota 55133-3427 (US)
(72) Inventor: HIRSCHBERG, Markus E., 41453 Neuss (DE); HINTZER, Klaus, 41453 Neuss (DE); LAMANNA, William M., Saint Paul, Minnesota 55133-3427 (US); SMITH, Sean M., Saint Paul, Minnesota 55133-3427 (US); RÖSCHENTHALER, Gerd-Volker, 28759 Bremen (DE); PAJKERT, Romana, 28759 Bremen (DE); TVERDOMED, Sergey N., 28759 Bremen (DE)
(74) Representative: Hettstedt, Stephan
(86) International application number: PCT/IB2018/060386
(87) International publication number: WO 2019/123342

(56) References cited:
- GUIZARD C. ET AL.: "Oxidative cleavage of partially or perfluorinated olefins by ruthenium tetroxide", JOURNAL OF FLUORINE CHEMISTRY, vol. 13, 1 January 1979 (1979-01-01), pages 175-177, XP002790055,
- BATTAIS A. ET AL.: "Synthèse d'acides perfluorés de hauts poids moléculaires", JOURNAL OF FLUORINE CHEMISTRY, vol. 19, 1 January 1981 (1981-01-01), pages 35-42, XP002790056,
- GORE E. S.: "Ruthenium catalysed oxidations of organic compounds", PLATINUM METALS REVIEW, vol. 27, no. 3, 1 January 1983 (1983-01-01), pages 111-125, XP002790057,

## Description

### Background

Fluoroelastomers are known to have excellent mechanical properties, heat resistance, weather resistance, and chemical resistance, for example. Such beneficial properties render fluoroelastomers useful, for example, as O-rings, seals, hoses, skid materials, and coatings (e.g., metal gasket coating for automobiles) that may be exposed to elevated temperatures or corrosive environments. Fluoroelastomers have been found useful in the automotive, chemical processing, semiconductor, aerospace, and petroleum industries, among others.

Fluoroelastomers are typically prepared by combining an amorphous fluoropolymer, sometimes referred to as a fluoroelastomer gum, with one or more curatives, shaping the resulting curable composition into a desired shape, and curing the curable composition. The amorphous fluoropolymer often includes a cure site, which is a functional group incorporated into the amorphous fluoropolymer backbone capable of reacting with a certain curative.

While cure sites have been introduced into amorphous fluoropolymers using, for example, difunctional organic chain transfer agents (such as CF₂I₂ or ICF₂CF₂CF₂CF₂I), and/or fluorinated cure site monomers having more than one functional group, these fluorinated compounds are sometimes expensive and challenging to synthesize.

Fluorinated carboxylic acids and their derivatives can be useful precursors to a variety of functional fluorinated compounds. Fluorinated carboxylic acids and their derivatives can be made, for example, by oxidative cleavage of fluorinated olefins. Prior methods of oxidizing fluorinated compounds utilized Lewis Acids, for example antimony pentafluoride and titanium chloride. Such methods provided low yields that are not workable for the production of usable quantities. Later methods added increased pressure, but still did not obtain desired yields. Other methods obtained higher yields, but utilized very large amounts of reagents (e.g., KMnO₄) and produced large amounts of undesirable byproducts. Methods described recently in Int. Pat. Appl. Pub. No. WO 2017/112445 (Hirschberg et al.) can require high temperatures, high pressures, and the use of gaseous starting materials in some cases, each of which can provide challenges.

CHRISTIAN GUIZARD et al.(Journal of Fluorine Chemistry, 13 (1979) 175-177) discloses oxidation of olefins. Ruthenium tetroxide is studied as oxidant. It gives cleavage products to form various alkenes.

A.BAITAIS et al. (*Journal of Fluorine* Chemistry, 19 (19111/82) 35-42) discloses perfluorinated olefins of general formula CF₃-(CF₂)ₚ -CF=CF-(CF₂)ₘ₋CF₃ (3≤m + p ≤25) oxidized by potassium permanganate or ruthenium tetroxide.

Ernest S. Gore (Platinum Metals Rev. 1983, 27, (3), 111-125) discloses that ruthenium and its complexes can be used to catalyse the oxidation of a range of organic substances including olefins.

### Summary

The present disclosure provides synthetic methods for making polyfunctional polyfluorinated compounds including an oxidative cleavage reaction that can be carried out at relatively low temperature and pressure. The oxidative cleavage reaction is typically high yielding for the desired polyfluorinated dicarboxylic acid and forms a low amount of side products.

In one aspect, the present disclosure provides a method of making a polyfunctional polyfluorinated compound that includes combining first components that include a polyfluorinated cyclic compound having 4 to 7 ring carbon atoms and ruthenium tetroxide wherein the first components comprise the precursor of ruthenium tetroxide wherein the precursor reacts with an oxidant to form ruthenium tetroxide, and wherein the precursor of the ruthenium tetroxide comprises ruthenium(IV) oxide hydrate, ruthenium(III) chloride hydrate, or a combination thereof and wherein the oxidant is a periodic acid or salt thereof. The precursor reacts with an oxidant to form ruthenium tetroxide. Two of the ring carbon atoms of the polyfluorinated cyclic compound form a double bond or an epoxide. Other ring carbon atoms of the polyfluorinated cyclic compound may be unsubstituted or substituted by polyfluoroalkyl groups.

In some embodiments, the method further includes converting the polyfluorinated dicarboxylic acid to a polyfluorinated dicarboxylic acid halide.

In some embodiments, the method further includes combining second components comprising the polyfluorinated dicarboxylic acid halide with a metal iodide to provide a polyfluorinated diiodide as the polyfunctional polyfluorinated compound.

In some embodiments, the polyfluorinated dicarboxylic acid halide is a polyfluorinated dicarboxylic acid fluoride, and the method further includes combining third components comprising the polyfluorinated dicarboxylic acid fluoride and hexafluoropropylene oxide to provide a polyfluorinated divinyl ether as the polyfunctional polyfluorinated compound.

In some embodiments, the polyfluorinated dicarboxylic acid halide is a polyfluorinated dicarboxylic acid fluoride, and the method further includes combining fourth components comprising the polyfluorinated dicarboxylic acid fluoride, fluoride ion, and CF₂=CF-CF₂-X, wherein X is Cl, Br, chlorosulfate, fluorosulfate, or trifluoromethyl sulfate, to provide a polyfluorinated diallyl ether as the polyfunctional polyfluorinated compound.

In this application:
Terms such as "a", "an" and "the" are not intended to refer to only a singular entity, but include the general class of which a specific example may be used for illustration. The terms "a", "an", and "the" are used interchangeably with the term "at least one".
The phrase "comprises at least one of' followed by a list refers to comprising any one of the items in the list and any combination of two or more items in the list. The phrase "at least one of' followed by a list refers to any one of the items in the list or any combination of two or more items in the list.
"Alkyl group" and the prefix "alk-" are inclusive of both straight chain and branched chain groups and of cyclic groups. Unless otherwise specified, alkyl groups herein have up to 20 carbon atoms. Cyclic groups can be monocyclic or polycyclic and, in some embodiments, have from 3 to 10 ring carbon atoms.
The terms "aryl" and "arylene" as used herein include carbocyclic aromatic rings or ring systems, for example, having 1, 2, or 3 rings and optionally containing at least one heteroatom (e.g., 0, S, or N) in the ring optionally substituted by up to five substituents including one or more alkyl groups having up to 4 carbon atoms (e.g., methyl or ethyl), alkoxy having up to 4 carbon atoms, halo (i.e., fluoro, chloro, bromo or iodo), hydroxy, or nitro groups. Examples of aryl groups include phenyl, naphthyl, biphenyl, fluorenyl as well as furyl, thienyl, pyridyl, quinolinyl, isoquinolinyl, indolyl, isoindolyl, triazolyl, pyrrolyl, tetrazolyl, imidazolyl, pyrazolyl, oxazolyl, and thiazolyl.
"Alkylene" is the multivalent (e.g., divalent or trivalent) form of the "alkyl" groups defined above. "Arylene" is the multivalent (e.g., divalent or trivalent) form of the "aryl" groups defined above.
"Arylalkylene" refers to an "alkylene" moiety to which an aryl group is attached. "Alkylarylene" refers to an "arylene" moiety to which an alkyl group is attached.
The term "polyfluorinated" refers to groups in which at least some C-H bonds are replaced by C-F bonds.
The terms "perfluoro" and "perfluorinated" refer to groups in which all C-H bonds are replaced by C-F bonds.

The term polyfunctional refers to having more than one functional group on the polyfluoroalkyl or perfluoroalkyl backbone. In some embodiments, polyfunctional refers to difunctional. Useful functional groups include carboxylic acids and their derivatives, vinyl ethers, allyl ethers, cyano groups, olefins, and halogens other than fluorine. In a polyfunctional (e.g., difunctional) group, the multiple functional groups need not be the same.

The phrase "interrupted by at least one -O- group", for example, with regard to a perfluoroalkyl or perfluoroalkylene group refers to having part of the perfluoroalkyl or perfluoroalkylene on both sides of the -O- group. For example, -CF₂CF₂-O-CF₂-CF₂- is a perfluoroalkylene group interrupted by an -0-.

All numerical ranges are inclusive of their endpoints and nonintegral values between the endpoints unless otherwise stated (e.g., 1 to 5 includes 1, 1.5, 2, 2.75, 3, 3.80, 4, 5, etc.).

### Detailed Description

The present disclosure provides a method of making a polyfunctional polyfluorinated compound. The method includes combining first components that include a polyfluorinated cyclic compound having 4 to 7 ring carbon atoms. The polyfluorinated cyclic compounds can be represented, for example, by Formula I or II below, wherein m is 0, 1, 2, or 3 to make the 4- to 7-membered rings. "F" in the center of the ring generally refers to the ring being perfluorinated although the method of the present disclosure can also be carried out with compounds in which not all C-H bonds have been replaced by C-F bonds. Two of the ring carbon atoms form a double bond as in Formula I below or an epoxide as in Formula II below. One or more of the carbon atoms in the ring, other than those in the double bond or epoxide, can be substituted with one or more polyfluoroalkyl groups Rf. The Rf groups typically have up to four (in some embodiments, three or two) carbon atoms and may be perfluorinated. In some embodiments, each Rf group is trifluoromethyl. In formulas I and II, n is independently in a range from 0 to 4, 0 to 3, or 0 to 2. In some embodiments, n is 0.

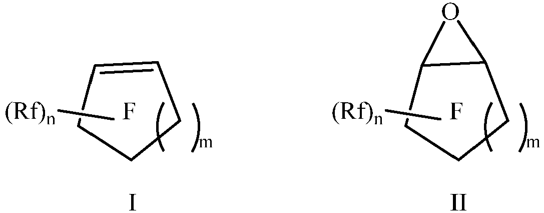

In some embodiments of the method of making a polyfunctional polyfluorinated compound, the polyfluorinated cyclic compound having 4 to 7 ring carbon atoms is a polyfluorinated cyclic olefin represented by formula I. Useful polyfluorinated cyclic olefins include partially fluorinated cyclobutene, hexafluorocyclobutene, partially fluorinated cyclopentene, octafluorocyclopentene, partially fluorinated cyclohexene, decafluorocyclohexene, partially fluorinated cycloheptene, and dodecafluorocycloheptene. In some embodiments, the polyfluorinated cyclic olefin is perfluorocyclopentene (i.e., octafluorocyclopentene). While these compounds are commercially available from chemical supply companies, we have found perfluorocyclopentene in a waste stream from the synthesis of a fluorinated olefin, which is an inexpensive source of supply.

The first components that are combined in the method of the present disclosure include ruthenium tetroxide (RuO₄) wherein the first components comprise the precursor of ruthenium tetroxide wherein the precursor reacts with an oxidant to form ruthenium tetroxide, and wherein the precursor of ruthenium tetroxide comprises ruthenium(IV) oxide hydrate, ruthenium(III) chloride hydrate, or a combination thereof and wherein the oxidant is a periodic acid or salt thereof In the presence of ruthenium tetroxide the polyfluorinated cyclic compound having 4 to 7 ring carbon atoms undergoes oxidative cleavage of the double bond or epoxide group to form a polyfluorinated dicarboxylic acid. An embodiment of the method is shown in Scheme I, below, in which an unsubstituted cyclic fluorinated olefin Ia is converted to a dicarboxylic acid X. In Scheme I, m and F are as defined above.

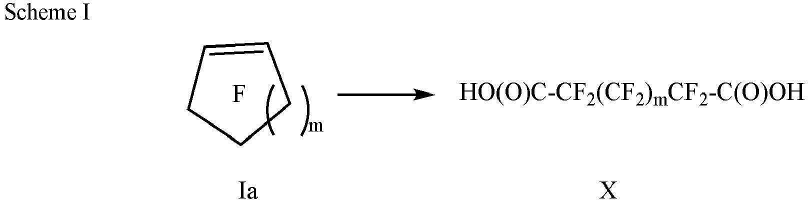

The first components comprise the precursor to ruthenium tetroxide. The precursor is a hydrate. The precursors to ruthenium tetroxide according to the invention comprises ruthenium(IV) oxide hydrate (RuO₂^{∗}H₂O) and ruthenium(III) chloride hydrate (RuCl₃^{∗}H₂O). Anhydrous RuO₂ was less efficient as a precursor for ruthenium tetroxide and, in some cases, did not generate ruthenium tetroxide as shown in the Examples, below. Precursors to ruthenium tetroxide are used under conditions in which ruthenium tetroxide is generated, for example, in the presence of a suitable oxidizing agent. Ruthenium(IV) oxide hydrate and ruthenium(III) chloride hydrate are readily oxidized by common oxidants (e.g. at least one of a perchlorate salt, a metaperiodate salt, a hypochlorite salt, orthoperiodic acid, or a salt thereof). Examples of oxidants include sodium metaperiodate, sodium hypochlorite, and orthoperiodic acid. According to the invention, the oxidant is a periodic acid or salt thereof (e.g., sodium metaperiodate or orthoperiodic acid). Oxidizing agents that do not readily oxidize ruthenium(IV) oxide hydrate and ruthenium(III) chloride hydrate include potassium bromate. In contrast to aqueous sodium hypochlorite without any further metal catalyst, ruthenium tetroxide strongly increases the regioselectivity of the ring-opening and strongly decreases the formation of by-products (e.g., difluoromalonic acid).

In some embodiments, the oxidant does not include an oxygen-containing gas. In these embodiments, the method is not carried out under conditions where an oxygen-containing gas is present. For example, although water can be considered an oxygen-containing gas under some conditions, the method is typically carried out at a temperature at which water is a liquid. An oxygen-containing gas can include oxygen (O₂), hydrogen peroxide (H₂O₂), ozone (O₃), nitrous oxide (N₂O), or combinations thereof.

The ruthenium tetroxide (RuO₄) or a precursor thereof is typically used in a catalytic amount. In some embodiments, a catalytic amount of the ruthenium tetroxide or a precursor thereof is up to 20 mole percent, up to 10 mole percent, up to 8 mole percent, up to 5 mole percent, or up to 1 mole percent, based on the total moles of the polyfluorinated cyclic compound in the first components. In some embodiments, a catalytic amount of the ruthenium tetroxide or a precursor thereof is at least 0.05 mole percent, based on the total moles of the polyfluorinated cyclic compound in the first components. In some embodiments, the amount of the ruthenium tetroxide or a precursor thereof is in a range from 0.05 mole percent to 5 mole percent or 0.1 mole percent to 1 mole percent, based on the total moles of the polyfluorinated cyclic compound in the first components.

The number of equivalents of oxidant in the first components comprising the precursor to ruthenium tetroxide is typically at least the same as the number of equivalents of the precursor and more typically greater than the number of equivalents of the precursor. In some embodiments, the number of moles of the oxidant (including any of those described above) to the number of moles of the precursor is at least 1 mole oxidant to 1 mole precursor (e.g., >1:1), up to 10 moles co-oxidant to 1 mole precursor (e.g., < 10:1). The ratio of oxidant to precursor can be up to 9:1, 8:1, 7:1, 6:1, or 5:1, in some embodiments.

Conveniently, reaction between the first components can be carried out at ambient temperature and pressure although higher temperature and pressure may be useful in some cases. In some embodiments, the first components are combined at ambient temperature (e.g., about 25 °C) or sub-ambient temperature. In some embodiments, the first components are combined at a temperature in a range from 0 °C to 25 °C or in a range from 0 °C to 10 °C. In some embodiments, the first components are allowed to react at a temperature of up 50 °C, less than 50 °C, up to 40 °C, up to 30 °C, or up to 25 °C. Conveniently, a temperature of at least 50 °C, at least 100 °C, or at least 400 °C, which is required for some transition catalyzed oxidations of cyclic fluorinated olefins (see, e.g., Int. App. Pub. No. WO 2017/112445 (Hirschberg et al.)), is not required for the method of the present disclosure. Useful reaction times include at least four hours and up to 72 hours, up to 48 hours, up to 24 hours, or up to 12 hours. Methods of the present disclosure can be carried out using known synthesis methods, processes, reaction vessels, and other standard equipment. The methods can be carried out in a batch mode, in a continuous mode (e.g., a flow reactor), or a combination thereof. The methods can be carried out in the bulk or in a solvent. Examples of suitable solvents include water and water-immiscible solvents such as halogenated solvents. The term "water-immiscible" refers to the solvent being incapable of being dissolved in water. While miscible solvents can dissolve in each other at any concentration, a solvent that is immiscible in water does not dissolved in water at every concentration. Examples of suitable halogenated solvents include carbon tetrachloride, hexachloroethane, 1,1,2-trichloro-1,2,2-trifluoroethane, C₅F₁₂, C₆F₁₄, C₇F₁₆, C₈F₁₈ and other perfluorocarbons (PFCs), perfluorinated hydrocarbons, perfluorinated amines, perfluorinated ethers, hydrofluorocarbons (e.g., from The Chemours Company, Wilmington, Del., under the trade designation "VERTREL"), and hydrofluoroethers such as methyl perfluorobutyl ether, ethyl perfluorobutyl ether, and those obtained from 3M Company, St. Paul, Minn., under the trade designation "NOVEC 7100" or "NOVEC 7200", and combinations thereof. Other fluorinated solvents (either partially fluorinated or perfluorinated) may also be useful. In some embodiments, the halogenated solvent is a hydrofluoroether. In some embodiments, the hydrofluoroether is represented by formula Rf³-[O-Rₕ]_{f}, wherein f is an integer from 1 to 3; Rf³ is a perfluoroalkyl or di- or trivalent perfluoroalkylene, each of which may be interrupted with at least one -O-, -S-, or -NH-; and Rₕ is an alkyl group optionally interrupted with at least one -O-, -S-, or -NH-. In some embodiments, when f is 1, Rf³ is selected from the group consisting of linear or branched perfluoroalkyl groups having from 2 to about 15 carbon atoms, perfluorocycloalkyl-containing perfluoroalkyl groups having from 5 to about 15 carbon atoms, and perfluorocycloalkyl groups having from 3 to about 12 carbon atoms. In some embodiments, when x is 2, Rf³ is selected from the group consisting of linear or branched perfluoroalkanediyl groups or perfluoroalkylidene groups having from 2 to about 15 carbon atoms, perfluorocycloalkyl- or perfluorocycloalkylene-containing perfluoroalkanediyl or perfluoroalkylidene groups having from 6 to about 15 carbon atoms, and perfluorocycloalkanediyl groups or perfluorocycloalkylidene groups having from 3 to about 12 carbon atoms. In some embodiments, when x is 3, Rf³ is selected from the group consisting of linear or branched perfluoroalkanetriyl groups having from 2 to about 15 carbon atoms, perfluorocycloalkyl- or perfluorocycloalkylene-containing perfluoroalkanetriyl groups having from 6 to about 15 carbon atoms, and perfluorocycloalkanetriyl groups having from 3 to about 12 carbon atoms. In some embodiments of formula Rf³-[O-Rₕ]_{f}, each Rₕ is independently selected from the group consisting of linear or branched alkyl groups having from 1 to about 8 carbon atoms, cycloalkyl-containing alkyl groups having from 4 to about 8 carbon atoms, and cycloalkyl groups having from 3 to about 8 carbon atoms. In some embodiments, the sum of the number of carbon atoms in Rf³ and the number of carbon atoms in Rₕ is greater than or equal to 4. The perfluorocycloalkyl and perfluorocycloalkylene groups contained within the perfluoroalkyl, perfluoroalkanediyl, perfluoroalkylidene and perfluoroalkanetriyl groups can optionally (and independently) be substituted with, e.g., one or more perfluoroalkyl groups having from 1 to about 4 carbon atoms. Suitable solvents have a boiling point in a range from about 25 °C to 200 °C. In some embodiments, the first components comprise an organic co-solvent, which may be miscible or immiscible with water. In some embodiments, the co-solvent includes a cyano group. Examples of suitable co-solvents include acetonitrile, propionitrile, adiponitrile, and combinations thereof. Combinations of any of these solvents may also be useful.

In some embodiments, the method of the present disclosure further includes converting the polyfluorinated dicarboxylic acid described above in any of its embodiments to a polyfluorinated dicarboxylic acid halide. The dicarboxylic acid halide may be a dicarboxylic acid chloride or a dicarboxylic acid fluoride. In some embodiments, the dicarboxylic acid halide is a dicarboxylic acid chloride, and the polyfunctional polyfluorinated compound made by the method of the present disclosure is a dicarboxylic acid chloride. The polyfluorinated dicarboxylic acid can be converted in a polyfluorinated dicarboxylic acid chloride using conventional methods (e.g. reacting with oxalylchloride, thionylchloride, or benzotrichloride). The conversion can be conveniently carried out by combining the polyfluorinated dicarboxylic acid with benzotrichloride in the presence of catalytic iron (III) chloride. The reaction can be carried out at elevated temperature, either neat or in the presence of a suitable solvent, and the product can be isolated by conventional methods (e.g., distillation). Similarly, the polyfluorinated dicarboxylic acid can be converted in a polyfluorinated dicarboxylic acid fluoride using conventional methods. The conversion can be conveniently carried out by combining the polyfluorinated dicarboxylic acid with benzotrifluoride in the presence of catalytic iron (III) chloride. The reaction can be carried out at elevated temperature, either neat or in the presence of a suitable solvent, and the product can be isolated by conventional methods (e.g., distillation).

Polyfluorinated dicarboxylic acid chlorides and polyfluorinated dicarboxylic acid fluorides can be converted to polyfluorinated dihalogenides (e.g. by the reaction with metal iodides). In this embodiment, the method of the present disclosure further comprises combining second components comprising the polyfluorinated dicarboxylic acid halide with a metal iodide to provide a polyfluorinated diiodide as the polyfunctional polyfluorinated compound. The reaction can be conveniently carried out by combining a polyfluorinated dicarboxylic acid chloride with lithium iodide, sodium iodide, potassium iodide, magnesium iodide, or calcium iodide or combining a polyfluorinated dicarboxylic acid fluoride and lithium iodide, magnesium iodide, or calcium iodide in an inert environment. The reaction can be carried out at elevated temperature, and the product can be carried out by conventional methods. An embodiment of a polyfluorinated dicarboxylic acid chloride XV made from polyfluorinated dicarboxylic acid X, described above, being converted into a diiodide XX is shown in Reaction Scheme II, below, wherein m is as defined above. In other embodiments, at least one of the carbons in the (CF₂)ₘ chain may be substituted with an Rf group as described above. Diiodides such as those shown in Reaction Scheme II can be useful, for example, as chain transfer agents in the preparation of amorphous fluorinated polymers and can introduce a cure site into the amorphous fluoropolymer. Examples of useful compounds of formula XX include I-(CF₂)₂-I, I-(CF₂)₃-I, I-(CF₂)₄-I, and I-(CF₂)₅-I.

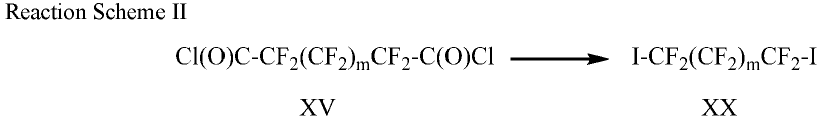

Polyfluorinated dicarboxylic acid fluorides can be converted to polyfluorinated divinyl ethers, for example, by reaction with hexafluoropropylene oxide in the presence of fluoride ion. In this embodiment, the method of the present disclosure further comprises combining third components comprising the polyfluorinated dicarboxylic acid fluoride and hexafluoropropylene oxide to provide a polyfluorinated divinyl ether as the polyfunctional polyfluorinated compound. The reaction can be conveniently carried out by combining the polyfluorinated dicarboxylic acid fluoride, hexafluoropropylene, and fluoride ion at a temperature in the range of -40 °C to 60 °C depending on the catalyst used, in non-reactive organic solvents that have a boiling point up to 275 °C (e.g. triglyme and tetraglyme), in some embodiments, having a boiling point up to 170 °C (e.g. CH₃CN, THF, propionitrile, monoglyme, diglyme) or mixtures thereof. The molar ratio of the halogenated fluoroorganyl diacid difluoride to HFPO is in the range of 1:1 to 1:10, in some embodiments, in the range of 1:2 to 1:5. The fluoride ion can be provided by a fluoride salt. In some embodiments, the source of the fluoride ion is at least one of sodium fluoride, potassium fluoride, rubidium fluoride, cesium fluoride, or (R)₄NF, wherein is each R is independently alkyl having from 1 to 6 carbon atoms, in some embodiments, 1 to 4 or 2 to 4 carbon atoms. An embodiment of a polyfluorinated dicarboxylic acid fluoride XVI made from polyfluorinated dicarboxylic acid X, described above, being converted into a divinyl ether XXV is shown in Reaction Scheme III, below, wherein m is as defined above. In other embodiments, at least one of the carbons in the (CF₂)ₘ chain may be substituted with an Rf group as described above. Examples of useful compounds of formula XXV include CF₂=CF-O-(CF₂)₄-O-CF=CF₂, CF₂=CF-O-(CF₂)₅-O-CF=CF₂, CF₂=CF-O-(CF₂)₆-O-CF=CF₂, and CF₂=CF-O-(CF₂)₇-O-CF=CF₂.

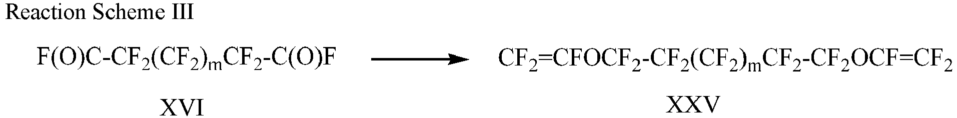

Polyfluorinated dicarboxylic acid fluorides can be converted to polyfluorinated diallyl ethers, for example, by reaction with CF₂=CF-CF₂-X, wherein X is a leaving group, in the presence of fluoride ion. In this embodiment, the method of the present disclosure further comprises combining fourth components comprising the polyfluorinated dicarboxylic acid fluoride, fluoride ion, and CF₂=CF-CF₂-X, wherein X is Cl, Br, chlorosulfate, fluorosulfate, or trifluoromethyl sulfate, to provide a polyfluorinated diallyl ether as the polyfunctional polyfluorinated compound. An embodiment of a polyfluorinated dicarboxylic acid fluoride XVI made from polyfluorinated dicarboxylic acid X, described above, being converted into a diallyl ether XXX is shown in Reaction Scheme IV, below, wherein m is as defined above. In other embodiments, at least one of the carbons in the (CF₂)ₘ chain may be substituted with an Rf group as described above. Divinyl ethers and diallyl ethers such as those shown in Reaction Schemes III and IV can be useful, for example, for introducing long-chain branching during the preparation of amorphous fluorinated polymers as described in U.S. Pat. Appl. Pub. No. 2010/0311906 (Lavallée et al.) and/or can introduce a cure site into the amorphous fluoropolymer.

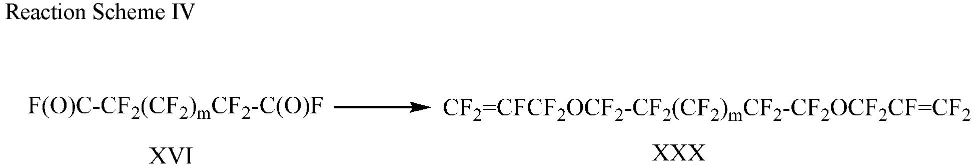

Compounds represented by XXX can be made, for example, by reacting dicarboxylic acid fluoride represented by formula XVI with perfluoroallyl chloride, perfluoroallyl bromide, or perfluoroallyl fluorosulfate in the presence of potassium fluoride as described in U.S. Pat. No. 4,273,729 (Krespan). Compounds represented by formula XXX can also be prepared by combining dicarboxylic acid fluoride represented by formula XVI, at least one of CF₂=CF-CF₂-OSO₂Cl or CF₂=CF-CF₂-OSO₂CF₃, and fluoride ion. The fluoride ion can be provided by a fluoride salt. In some embodiments, the source of the fluoride ion is at least one of sodium fluoride, potassium fluoride, rubidium fluoride, cesium fluoride, or (R)₄NF, wherein is each R is independently alkyl having from 1 to 6 carbon atoms, in some embodiments, 1 to 4 or 2 to 4 carbon atoms. Suitable solvents for the transformation include polar, aprotic solvents, for example, N,N-dimethylformamide (DMF), acetonitrile, N,N-dimethylacetamide (DMAC), .gamma-butyrolactone, 1,2-dimethoxyethane (glyme), 1-(2-methoxyethoxy)-2-methoxyethane (diglyme), 2,5,8,11-tetraoxadodecane (triglyme), dioxane, sulfolane, nitrobenzene, and benzonitrile.

CF₂=CF-CF₂-OSO₂Cl can conveniently be prepared by reaction of boron trichloride (BCl₃) and ClSO₃H to provide B(OSO₂Cl)₃ and subsequently reacting the B(OSO₂Cl)₃ and hexafluoropropylene (HFP). The reaction of BCl₃ and ClSO₃H can be carried out, for example, by dropwise addition of neat ClSO₃H to gaseous BCl₃ at below 50 °C or, in the case of condensed BCl₃ at sub-ambient temperature. The reaction can be carried out at a temperature of least -20 °C, -10 °C, 0 °C, 10 °C, or 20 °C and up to 30 °C, 40 °C, or 50 °C. The addition of ClSO₃H to BCl₃ can be carried out at a rate, for example, to maintain the temperature of the mixture at 10 °C or below. B(OSO₂Cl)₃ can be isolated as a white powder after volatile starting materials are removed under vacuum. B(OSO₂Cl)₃ can then be suspended or dissolved in a solvent, and HFP can be added at below 50 °C, in some embodiments, at sub-ambient temperature. For example, the reaction can be carried out at a temperature of least -20 °C, -10 °C, 0 °C, 10 °C, or 20 °C and up to 30 °C, 40 °C, or 50 °C. Suitable solvents include halogenated solvents (e.g., methylene chloride or Freon-113). In some embodiments, the solvent is a non-aromatic solvent. CF₂=CF-CF₂-OSO₂Cl can be isolated and optionally purified using conventional methods.

Combining components comprising M(OSO₂CF₃)₃ and hexafluoropropylene (HFP) provides CF₂=CF-CF₂-OSO₂CF₃, wherein M is Al or B. Al(OSO₂CF₃)₃ is commercially available, for example, from chemical suppliers such as abcr GmbH (Karlsruhe, Germany) and Sigma-Aldrich (St. Louis, Missouri). Reaction of BCl₃ and CF₃SO₃H can be useful to provide B(OSO₂CF₃)₃. The reaction of BCl₃ and CF₃SO₃H can be carried out, for example, by dropwise addition of neat CF₃SO₃H to gaseous BCl₃ at below 50 °C or, in the case of condensed BCl₃ at sub-ambient temperature. The reaction can be carried out at a temperature of least -20 °C, -10 °C, 0 °C, 10 °C, or 20 °C and up to 30 °C, 40 °C, or 50 °C. The addition of CF₃SO₃H to BCl₃ can be carried out at a rate, for example, to maintain the temperature of the mixture at 10 °C or below. B(OSO₂CF₃)₃ can be isolated as a white powder after volatile starting materials are removed under vacuum.

B(OSO₂CF₃)₃ can be combined with HFP at a temperature above 0 °C. In some embodiments, the reaction can be carried out at a temperature up to 50 °C, 40 °C, 30 °C, 20 °C, or 10 °C. The reaction can be carried out at a temperature in a range from above 0 °C to 10 °C, in some embodiments, in a range from 2 °C to 10 °C, and in some embodiments, in a range from 4 °C to 8 °C. The reaction mixture is combined with water at a temperature below 28 °C, in some embodiments, in a range from above 25 °C to 27 °C. The reaction product can then be isolated and optionally purified using conventional methods (e.g., separation of the organic fraction, drying over a drying agent, filtering, and distilling). The product CF₂=CF-CF₂-OSO₂CF₃ can be isolated in 75% yield, which is an improvement over the yield reported in Petrov, V. A., J. Fluorine Chem. 1995, 73, 17-19.

Combinations of the methods shown in Reaction Schemes II, III, and IV can be useful, for example, for making cure site monomers, in which one of acid fluoride groups is converted to a polyfluorinated vinyl ether or allyl ether, and the other of the acid fluoride groups is converted to an iodide. Such cure site monomers can be useful, for example, in the preparation of amorphous fluorinated polymers and can introduce a cure site into the amorphous fluoropolymer. One or more of the fluorinated divinyl ethers in a compound of formula XXV can be iodinized with a mixture of elemental iodine and iodine pentafluoride at a temperature in the range of 30 °C to 200 °C, or in the range of 80 °C to 160 °C. Alternatively, the reaction of the resulting divinyl ether could be conducted with ICl, HF, and BF₃ at about 50 °C to form further polyfunctional polyfluorinated compounds. Examples of such compounds include I-CF₂-CF₂-O-(CF₂)₄-O-CF=CF₂, I-CF₂-CF₂-O-(CF₂)₄-O-CF₂-CF₂-I, I-CF₂-CF₂-O-(CF₂)₅-O-CF=CF₂,I-CF₂-CF₂-O-(CF₂)₅-O-CF₂-CF₂-I,I-CF₂-CF₂-O-(CF₂)₆-O-CF=CF₂, I-CF₂-CF₂-O-(CF₂)₆-O-CF₂-CF₂-I, I-CF₂-CF₂-O-(CF₂)₇-O-CF=CF₂ and I-CF₂-CF₂-O-(CF₂)₇-O-CF₂-CF₂-I.

Polyfluorinated dicarboxylic acid fluorides can also be converted into cure site monomers as shown in Reaction Scheme V below, where m is as defined above. In other embodiments, at least one of the carbons in the (CF₂)ₘ chain may be substituted with an Rf group as described above. In one embodiment, one of the acid fluoride groups in a compound of formula XVI can be converted to a polyfluorinated vinyl ether, for example, by reaction with one equivalent hexafluoropropylene oxide in the presence of fluoride ion using the method described above. The other of the acid fluoride groups can be converted into a cyano group by known methods [e.g., esterification (e.g. with CH₃OH), amination and subsequent oxidation (e.g. with P₄O₁₀) as described, for example, in EP0710645A1 (1996) and EP0708139A1 (1996)]. Cure site monomers such as XXXV shown in Reaction Scheme V can be useful, for example, in the preparation of amorphous fluorinated polymers and can introduce a cure site into the amorphous fluoropolymer. Examples of useful compounds of formula XXXV include CF₂=CF-O-(CF₂)₃-CN, CF₂=CF-O-(CF₂)₄-CN, CF₂=CF-O-(CF₂)₅-CN and CF₂=CF-O-(CF₂)₆-CN.

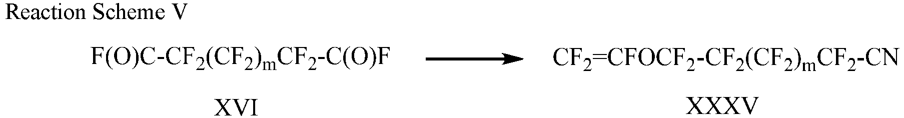

A compound of formula XX can also be converted into a cure site monomer (for example by adding ethylene and consecutive dehydrohalogenation to the corresponding olefin) for subsequent crosslinking by known methods described in literature. The reaction can be carried out as described, for example, in Bull. Acad. Sci. USSR, Div. Chem. Sci. 1964, 359-361 and J. Org. Chem. 1977, 42, 1985-1990. Useful compounds that can be made by this method include I-(CF₂)x-CH=CH₂ and CH₂=CH-(CF₂)x-CH=CH₂, in which x is 2, 3, 4, or 5.

For the reaction of polyfluorinated dicarboxylic acid fluoride such as XVI, one or two equivalents of components other than the polyfluorinated dicarboxylic acid fluoride may be useful for converting the polyfluorinated dicarboxylic acid fluoride into a polyfunctional polyfluorinated compound in which the functional groups are different or the same, respectively. The desired number of equivalents of components other than the polyfluorinated dicarboxylic acid fluoride may be exceeded in some cases by up to 10 mol%, 7.5 mol%, or 5 mol%.

Polyfunctional polyfluorinated compounds made according to the methods of the present disclosure are useful, for example, in the preparation of fluoropolymers. For example, polyfunctional polyfluorinated compounds can be interpolymerized with at least one partially fluorinated or perfluorinated ethylenically unsaturated monomer represented by formula R^{a}CF=CR^{a}₂, wherein each R^{a} is independently fluoro, chloro, bromo, hydrogen, a fluoroalkyl group (e.g. perfluoroalkyl having from 1 to 8, 1 to 4, or 1 to 3 carbon atoms and optionally interrupted by one or more oxygen atoms), fluoroalkoxy group (e.g. perfluoroalkoxy having from 1 to 8, 1 to 4, or 1 to 3 carbon atoms and optionally interrupted by one or more oxygen atoms), alkyl having up to 10 carbon atoms, alkoxy having up to 8 carbon atoms, or aryl having up to 8 carbon atoms. Examples of useful fluorinated monomers represented by formula R^{a}CF=CR^{a}₂ include vinylidene fluoride (VDF), tetrafluoroethylene (TFE), hexafluoropropylene (HFP), chlorotrifluoroethylene, 2-chloropentafluoropropene, trifluoroethylene, vinyl fluoride, dichlorodifluoroethylene, 1,1-dichlorofluoroethylene, 1-hydropentafluoropropylene, 2-hydropentafluoropropylene, tetrafluoropropylene, perfluoroalkyl perfluorovinyl ethers, perfluoroalkyl perfluoroallyl ethers, and mixtures thereof.

In some embodiments, the fluoropolymer that includes units derived from a polyfunctional polyfluorinated compound made by the present disclosure includes units from one or more monomers independently represented by formula CF₂=CFORf, wherein Rf is perfluoroalkyl having from 1 to 8, 1 to 4, or 1 to 3 carbon atoms, optionally interrupted by one or more -O- groups. Perfluoroalkoxyalkyl vinyl ethers suitable for making a fluoropolymer include those represented by formula CF₂=CF(OCₙF₂ₙ)_{z}ORf₂, in which each n is independently from 1 to 6, z is 1 or 2, and Rf₂ is a linear or branched perfluoroalkyl group having from 1 to 8 carbon atoms and optionally interrupted by one or more -O- groups. In some embodiments, n is from 1 to 4, or from 1 to 3, or from 2 to 3, or from 2 to 4. In some embodiments, n is 1 or 3. In some embodiments, n is 3. CₙF₂ₙ may be linear or branched. In some embodiments, CₙF₂ₙ can be written as (CF₂)ₙ, which refers to a linear perfluoroalkylene group. In some embodiments, CₙF₂ₙ is -CF₂-CF₂-CF₂-. In some embodiments, CₙF₂ₙ is branched, for example, -CF₂-CF(CF₃)-. In some embodiments, (OCₙF₂ₙ)_{z} is represented by -O-(CF₂)₁₋₄-[O(CF₂)₁₋₄]₀₋₁. In some embodiments, Rf₂ is a linear or branched perfluoroalkyl group having from 1 to 8 (or 1 to 6) carbon atoms that is optionally interrupted by up to 4, 3, or 2 -O- groups. In some embodiments, Rf₂ is a perfluoroalkyl group having from 1 to 4 carbon atoms optionally interrupted by one -O- group. Suitable monomers represented by formula CF₂=CFORf and CF₂=CF(OCₙF₂ₙ)_{z}ORf₂ include perfluoromethyl vinyl ether, perfluoroethyl vinyl ether, perfluoropropyl vinyl ether, CF₂=CFOCF₂OCF₃, CF₂=CFOCF₂OCF₂CF₃, CF₂=CFOCF₂CF₂OCF₃, CF₂=CFOCF₂CF₂CF₂OCF₃, CF₂=CFOCF₂CF₂CF₂CF₂OCF₃, CF₂=CFOCF₂CF₂OCF₂CF₃, CF₂=CFOCF₂CF₂CF₂OCF₂CF₃, CF₂=CFOCF₂CF₂CF₂CF₂OCF₂CF₃, CF₂=CFOCF₂CF₂OCF₂OCF₃, CF₂=CFOCF₂CF₂OCF₂CF₂OCF₃, CF₂=CFOCF₂CF₂OCF₂CF₂CF₂OCF₃, CF₂=CFOCF₂CF₂OCF₂CF₂CF₂CF₂OCF₃, CF₂=CFOCF₂CF₂OCF₂CF₂CF₂CF₂CF₂OCF₃, CF₂=CFOCF₂CF₂(OCF₂)₃OCF₃, CF₂=CFOCF₂CF₂(OCF₂)₄OCF₃, CF₂=CFOCF₂CF₂OCF₂OCF₂OCF₃, CF₂=CFOCF₂CF₂OCF₂CF₂CF₃ CF₂=CFOCF₂CF₂OCF₂CF₂OCF₂CF₂CF₃, CF₂=CFOCF₂CF(CF₃)-O-C₃F₇ (PPVE-2), CF₂=CF(OCF₂CF(CF₃))₂-O-C₃F₇ (PPVE-3), and CF₂= CF(OCF₂CF(CF₃))₃-O-C₃F₇ (PPVE-4). Many of these perfluoroalkoxyalkyl vinyl ethers can be prepared according to the methods described in U.S. Pat. Nos. 6,255,536 (Worm et al.) and 6,294,627 (Worm et al.).

Perfluoroalkyl alkene ethers and perfluoroalkoxyalkyl alkene ethers may also be useful in the preparation of a fluoropolymer that includes units derived from a polyfunctional polyfluorinated compound made by the present disclosure. In addition, the fluoropolymers may include interpolymerized units of fluoro (alkene ether) monomers, including those described in U.S. Pat. Nos. 5,891,965 (Worm et al.) and 6,255,535 (Schulz et al.). Such monomers include those represented by formula CF₂=CF(CF₂)ₘ-O-R_{f}, wherein m is an integer from 1 to 4, and wherein R_{f} is a linear or branched perfluoroalkylene group that may include oxygen atoms thereby forming additional ether linkages, and wherein R_{f} contains from 1 to 20, in some embodiments from 1 to 10, carbon atoms in the backbone, and wherein R_{f} also may contain additional terminal unsaturation sites. Suitable perfluoroalkoxyalkyl allyl ethers include those represented by formula CF₂=CFCF₂(OCₙF₂ₙ)_{z}ORf₂, in which n, z, and Rf₂ are as defined above in any of the embodiments of perfluoroalkoxyalkyl vinyl ethers. Examples of suitable perfluoroalkoxyalkyl allyl ethers include CF₂=CFCF₂OCF₂CF₂OCF₃, CF₂=CFCF₂OCF₂CF₂CF₂OCF₃, CF₂=CFCF₂OCF₂OCF₃, CF₂=CFCF₂OCF₂OCF₂CF₃, CF₂=CFCF₂OCF₂CF₂CF₂CF₂OCF₃, CF₂=CFCF₂OCF₂CF₂OCF₂CF₃, CF₂=CFCF₂OCF₂CF₂CF₂OCF₂CF₃, CF₂=CFCF₂OCF₂CF₂CF₂CF₂OCF₂CF₃, CF₂=CFCF₂OCF₂CF₂OCF₂OCF₃, CF₂=CFCF₂OCF₂CF₂OCF₂CF₂OCF₃, CF₂=CFCF₂OCF₂CF₂OCF₂CF₂CF₂OCF₃, CF₂=CFCF₂OCF₂CF₂OCF₂CF₂CF₂CF₂OCF₃, CF₂=CFCF₂OCF₂CF₂OCF₂CF₂CF₂CF₂CF₂OCF₃, CF₂=CFCF₂OCF₂CF₂(OCF₂)₃OCF₃, CF₂=CFCF₂OCF₂CF₂(OCF₂)₄OCF₃, CF₂=CFCF₂OCF₂CF₂OCF₂OCF₂OCF₃, CF₂=CFCF₂OCF₂CF₂OCF₂CF₂CF₃, CF₂=CFCF₂OCF₂CF₂OCF₂CF₂OCF₂CF₂CF₃, CF₂=CFCF₂OCF₂CF(CF₃)-O-C₃F₇, and CF₂=CFCF₂(OCF₂CF(CF₃))₂-O-C₃F₇. Many of these perfluoroalkoxyalkyl allyl ethers can be prepared, for example, according to the methods described in U.S. Pat. No. 4,349,650 (Krespan).

Perfluoro-1,3-dioxoles may also be useful to prepare a fluoropolymer that includes units derived from a polyfunctional polyfluorinated compound made by the present disclosure. Perfluoro-1,3-dioxole monomers and their copolymers are described in U. S. Pat. No. 4,558, 141 (Squire).

Polyfunctional polyfluorinated compounds can be useful for preparing amorphous fluoropolymers, semi-crystalline thermoplastics, and non-melt processable fluoroplastics.

In some embodiments, one or more polyfunctional polyfluorinated compounds can be copolymerized with TFE to form a non-melt processable fluoroplastic. The polyfunctional polyfluorinated compound may be any of those described above. In a non-melt processable fluoroplastic, one or more polyfunctional polyfluorinated compounds are included in the monomers for polymerization in an amount of up to about one percent by weight. TFE homo-and copolymers including a comonomer in an amount of up to about one percent by weight are referred to in the art as PTFE. PTFE has such a high melt viscosity and/or low melt flow index (MFI) that it cannot be processed by conventional melt processing techniques such as extrusion, injection molding, or blow molding. In some embodiments, the fluoropolymer contains TFE units and units from at least one polyfunctional polyfluorinated compound and no other comonomer units. The amount of the polyfunctional polyfluorinated compound comonomer units may be up to 1% by weight or up to 0.1% by weight. For example, the amount of the polyfunctional polyfluorinated compound comonomer units can be from 0.01 to 1 percent by weight or from 0.3 to 1 percent by weight, based on the total weight of the fluoropolymer (in which the comonomer units add up to give 100% by weight).

The molecular weights of certain fluoroplastics are often characterized by the melt viscosity or the melt flow index (MFI; e.g., 372 °C/5 kg). In some embodiments, the non-melt-processable fluoropolymer made from the polyfunctional polyfluorinated compound has a melt flow index (MFI) of 1.0 g / 10 min or less at 372 °C using a 5 kg load (MFI 372/5 of less than 1.0 g /10 min), in some embodiments, a melt flow index (372/5) of 0.1 g /10 minutes or less. In some embodiments, the non-melt-processable fluoropolymer has a melting point of at least 300 °C, in some embodiments, at least 315 °C, and typically within the range of 327 +/-10 °C. In some embodiments, the non-melt-processable fluoropolymer has a melting point of at least 317 °C, at least 319 °C, or at least 321 °C. The melting point of not melt-processable fluoropolymers differs when the material is molten for the first time and after subsequent melting. After the material has been molten once, the meting point in subsequent melting remains constant. The melting point as referred to herein is the melting point of previously molten material (i.e., the material was brought to the melting point, cooled below its melting point, and then melted again).

PTFEs made with one or more polyfunctional polyfluorinated compounds made by the methods disclosed herein can be useful, for example, for gaskets and inner liners for pipes and containers.

In some embodiments, one or more polyfunctional polyfluorinated compound can be copolymerized with TFE to form a fluorothermoplastic. Copolymers of TFE and perfluorinated vinyl or allyl ethers are known in the art as PFA's (perfluorinated alkoxy polymers). In these embodiments, the fluorinated vinyl or allyl ether units are present in the copolymer in an amount in a range from 0.5 mol% to 15 mol%, in some embodiments, 0. 5 mol% to 10 mol%, and in some embodiments, 0.5 mol% to 5 mol%. The polyfunctional fluorinated allyl ether may be any of those described above. In some embodiments, the copolymer of TFE and at least one fluorinated vinyl ether or allyl ether consists essentially of units derived from TFE and at least one polyfunctional polyfluorinated compound. "Consisting essentially of' as used herein refers to the absence of other comonomers or the presence of units derived from other comonomers in an amount of less than one percent by weight, in some embodiments, less than 0.1 percent by weight. In some embodiments, the copolymer of TFE and at least one polyfunctional polyfluorinated compound further comprises at least one percent by weight, in some embodiments, up to 10, 6, 5, or 4 percent by weight of other units derived from compounds represented by formula R^{a}CF=CR^{a}₂ described above, non-fluorinated olefins (e.g., ethene or propene). In some embodiments, at least one of HFP, VDF, vinyl fluoride, chlorotrifluoroethylene, ethene, or propene is included in the monomers in an amount up to ten percent by weight to make the PFA copolymer. In some embodiments, the fluorothermoplastic made from the compound comprising at least one perfluorinated vinyl or allyl ether group has a melt flow index (MFI) in a range from 0.5 g / 10 min to 100 g / 10 min at 372 °C using a 5 kg load (MFI 372/5 of in a range from 0.5 g /10 min to 100 g / 10 min). In some embodiments, the copolymer has a melting point of from 270 °C to 326 °C and a melt flow index (MFI at 372 °C and 5 kg load) of 0.5 to 19 grams / 10 minutes. In some embodiments, the copolymer has a melting point of from 250 °C to 290 °C and have a melt flow index (MFI at 372 °C and 5 kg load) of from 31 grams / 10 minutes to 50 grams / 10 minutes.

In some embodiments, one or more polyfunctional polyfluorinated compounds can be copolymerized with TFE and HFP. The polyfunctional polyfluorinated compound may be any of those described above. Copolymers of TFE and HFP with or without other perfluorinated comonomers are known in the art as FEP's (fluorinated ethylene propylene). In some embodiments, these fluorothermoplastics are derived from copolymerizing 30 to 70 wt. % TFE, 10 to 30 wt. %, HFP, and 0.2 to 50 wt. % of other comonomers and one or more of the polyfunctional polyfluorinated compounds. These weight percentages are based on the weight of the polymer, and the comonomers add up to give 100% by weight. In some embodiments, units derived from the polyfunctional polyfluorinated compounds are present in the copolymer according to the present disclosure in a range from 0.2 percent by weight to 12 percent by weight, based on the total weight of the copolymer. In some embodiments, units derived from the polyfunctional polyfluorinated compound are present in a range from 0.5 percent by weight to 6 percent by weight, based on the total weight of the copolymer, with the total weight of the copolymer being 100% by weight. In some embodiments, units derived from the polyfunctional polyfluorinated compound are present in the copolymer according to the present disclosure in a range from 0.02 mole percent to 2 mole percent, based on the total amount of the copolymer. In some embodiments, units derived from the polyfunctional polyfluorinated compound are present in the copolymer in an amount up to 1.5 mole percent or up to 1.0 mole percent. In some embodiments, the copolymerized units derived from polyfunctional polyfluorinated compound are present in the copolymer in an amount of at least 0.03 mole percent or 0.05 mole percent. The copolymerized units derived from may be present in the copolymer in a range from 0.02 mole percent to 2 mole percent, 0.03 mole percent to 1.5 mole percent, or 0.05 mole percent to 1.0 mole percent. The HFP may be present in a range from 5 wt. % to 22 wt. %, in a range from 10 wt. % to 17 wt. %, in a range from 11 wt. % to 16 wt. %, or in a range from 11.5 wt. % to 15.8 wt. %, based on the total weight of the copolymer, wherein the weight of the copolymer is 100% by weight. The copolymers made according to the methods of the present disclosure typically have a melting point between 220 °C to 285 °C, in some embodiments, 235 °C to 275 °C, 240 °C to 275 °C, or 245 °C to 265 °C. In some embodiments, the copolymer prepared from the polyfunctional polyfluorinated compound, TFE, and HFP has an MFI at 372 °C and 5 kg load of 30 ± 10 grams per 10 minutes. In some embodiments, the copolymer prepared from the polyfunctional polyfluorinated compound, TFE, and HFP has an MFI at 372 °C and 5 kg load of 30 ± 5 grams per 10 minutes or 30 ± 3 grams per 10 minutes. In some embodiments, the copolymer prepared from the polyfunctional polyfluorinated compound, TFE, and HFP has an MFI at 372 °C and 5 kg load in a range from 1 gram per 10 minutes to 19 grams per 10 minutes. In some embodiments, this copolymer has an MFI in a range from 1 gram per 10 minutes to 15 grams per 10 minutes or in a range from 1 gram per 10 minutes to 10 grams per 10 minutes.

FEPs made with one or more polyfunctional polyfluorinated compounds made by the methods disclosed herein can be useful, for example, for electrical insulation in Local Area Networks (LAN).

In some embodiments, one or more polyfunctional polyfluorinated compounds made by the methods disclosed herein can be used to make amorphous fluoropolymers. Amorphous fluoropolymers typically do not exhibit a melting point and exhibit little or no crystallinity at room temperature. Useful amorphous fluoropolymers can have glass transition temperatures below room temperature or up to 280 °C. Suitable amorphous fluoropolymers can have glass transition temperatures in a range from -60 °C up to 280 °C, -60 °C up to 250 °C, from -60 °C to 150 °C, from -40 °C to 150 °C, from -40 °C to 100 °C, or from -40 °C to 20 °C.

In some embodiments, amorphous fluoropolymers that include units derived from a polyfunctional polyfluorinated compound prepared using the methods disclosed herein include a TFE/propylene copolymer, a TFE/propylene/VDF copolymer, a VDF/HFP copolymer, a TFE/VDF/HFP copolymer, a TFE/ perfluoromethyl vinyl ether (PMVE) copolymer, a TFE/CF₂=CFOC₃F₇ copolymer, a TFE/CF₂=CFOCF₃/CF₂=CFOC₃F₇ copolymer, a TFE/ethyl vinyl ether (EVE) copolymer, a TFE/butyl vinyl ether (BVE) copolymer, a TFE/EVE/BVE copolymer, a VDF/CF₂=CFOC₃F₇ copolymer, an ethylene/HFP copolymer, a TFE/ HFP copolymer, a CTFE/VDF copolymer, a TFE/VDF copolymer, a TFE/VDF/PMVE/ethylene copolymer, or a TFE/VDF/CF₂=CFO(CF₂)₃OCF₃ copolymer.

In some embodiments, the amorphous fluoropolymer that includes units derived from a polyfunctional polyfluorinated compound prepared using the methods disclosed herein includes polymerized units comprising a cure site. In these embodiments, cure site monomers (including any of those described above, for example, compounds of formula XXV, XXX, XXXV, and I-CF₂-CF₂-O-(CF₂)₄-O-CF=CF₂, I-CF₂-CF₂-O-(CF₂)₅-O-CF=CF₂, I-CF₂-CF₂-O-(CF₂)₆-O-CF=CF₂, and I-CF₂-CF₂-O-(CF₂)₇-O-CF=CF₂) may be useful during the polymerization to make the amorphous fluoropolymer. Such cure site monomers include those monomers capable of free radical polymerization. The cure site monomer can be perfluorinated to ensure adequate thermal stability of the resulting elastomer. Examples of useful cure sites include a Br cure site, an I cure site, a nitrile cure site, a carbon-carbon double bond, and combinations thereof. Any of these cure sites can be cured using peroxides, for example. However, in some cases in which multiple, different cure sites are present a dual cure system or a multi cure system may be useful. Other suitable cure systems that may be useful include bisphenol curing systems or triazine curing systems. Useful amounts of the cure site monomers include 0.01 mol % to 1 mol %, based on total moles of monomer incorporated into the polymer may be used. In some embodiments, at least 0.02, 0.05, or even 0.1 mol % of a cure site monomer is used and at most 0.5, 0.75, or even 0.9 mol % of a cure site monomer is used based on the total moles of monomer incorporated into the amorphous fluoropolymer.

In some embodiments, the amorphous fluoropolymer that includes units derived from a polyfunctional polyfluorinated compound prepared using the methods disclosed herein includes polymerized units comprising a nitrile cure site. Nitrile cure sites can be introduced into the polymer by using nitrile containing monomers during the polymerization. Examples of suitable nitrile containing monomers include those represented by formulas CF₂=CF-O-(CF₂)₄-CN and CF₂=CF-O-(CF₂)₅-CN, prepared as described above.

In some embodiments, the amorphous fluoropolymer that includes units derived from a polyfunctional polyfluorinated compound prepared using the methods disclosed herein includes polymerized units comprising at least one compound represented by XXV or XXX as a cure site monomer, as described above in any of its embodiments. The compound represented by formula XXV or XXX may be present in the components to be polymerized in any useful amount, in some embodiments, in an amount of up to 2, 1, or 0.5 mole percent and in an amount of at least 0.1 mole percent, based on the total amount of polymerizable components.

If the amorphous fluoropolymer is perhalogenated, in some embodiments perfluorinated, typically at least 50 mole percent (mol %) of its interpolymerized units are derived from TFE and/or CTFE, optionally including HFP. The balance of the interpolymerized units of the amorphous fluoropolymer (e.g., 10 to 50 mol %) is made up of one or more perfluorinated vinyl or allyl ethers, and the cure site monomer. If the fluoropolymer is not perfluorinated, it typically contains from about 5 mol % to about 90 mol % of its interpolymerized units derived from TFE, CTFE, and/or HFP; from about 5 mol % to about 90 mol % of its interpolymerized units derived from VDF, ethylene, and/or propylene; up to about 40 mol % of its interpolymerized units derived from perfluorinated vinyl or allyl ethers; and from about 0.1 mol % to about 5 mol %, in some embodiments from about 0.3 mol % to about 2 mol %, of the cure site monomer.

A diiodofluoroalkane and/or a diiodomethane can be useful as chain transfer agents. Examples of useful diiodo-fluoroalkane chain transfer agents include: I(CF₂)ₙI, where n is an integer from 2-5. Useful amounts of the chain transfer agents include 0.01 wt% up to 2 wt% based on the total amount of comonomer used. More information about chain transfer agents can be found, for example, in copending application PCT/US2017/059825, filed November 3, 2017.

Fluoropolymers that include units derived from the polyfunctional polyfluorinated compounds made by the method disclosed herein can be made by free-radical polymerization. Conveniently, in some embodiments, the methods of making the copolymer disclosed herein includes radical aqueous emulsion polymerization using a sequence of steps, which can include polymerization, coagulation, washing, and drying. In some embodiments, an aqueous emulsion polymerization can be carried out continuously under steady-state conditions. For example, an aqueous emulsion of monomers (e.g,. including any of those described above), water, emulsifiers, buffers and catalysts can be fed continuously to a stirred reactor under optimum pressure and temperature conditions while the resulting emulsion or suspension is continuously removed. In some embodiments, batch or semibatch polymerization is conducted by feeding the aforementioned ingredients into a stirred reactor and allowing them to react at a set temperature for a specified length of time or by charging ingredients into the reactor and feeding the monomers into the reactor to maintain a constant pressure until a desired amount of polymer is formed. After polymerization, unreacted monomers are removed from the reactor effluent latex by vaporization at reduced pressure. The fluoropolymer can be recovered from the latex by coagulation.

### Some Embodiments of the Disclosure

In a first embodiment, the present disclosure provides a method of making a polyfunctional polyfluorinated compound, the method comprising:
combining first components comprising:
   a polyfluorinated cyclic compound having 4 to 7 ring carbon atoms, wherein two of the ring carbon atoms form a double bond or an epoxide; and , ruthenium tetroxide and
forming a polyfluorinated dicarboxylic acid wherein the first components comprise the precursor of ruthenium tetroxide wherein the precursor reacts with an oxidant to form ruthenium tetroxide, and wherein the precursor of ruthenium tetroxide comprises ruthenium(IV) oxide hydrate, ruthenium(III) chloride hydrate, or a combination thereof and wherein the oxidant is a periodic acid or salt thereof.

In a second embodiment, the present disclosure provides the method of the first embodiment, wherein the polyfluorinated cyclic compound having 4 to 7 ring carbon atoms is substituted by one or more polyfluoroalkyl groups.

In a third embodiment, the present disclosure provides the method of the first or second embodiment, wherein the polyfluorinated cyclic compound having 4 to 7 ring carbon atoms is a polyfluorinated cyclic olefin.

In a fourth embodiment, the present disclosure provides the method of any one of the first to third embodiments, wherein the polyfluorinated cyclic olefin is perfluorocyclopentene.

In a fifth embodiment, the present disclosure provides the method of the fourth embodiment, further comprising obtaining perfluorocyclopentene from a waste stream.

In a tenth embodiment, the present disclosure provides the method of any one of the first to fifth embodiments, wherein the oxidant comprises a periodic acid or a salt thereof.

In an eleventh embodiment, the present disclosure provides the method of any one of the first to tenth embodiments, wherein the oxidant does not include an oxygen-containing gas.

In a twelfth embodiment, the present disclosure provides the method of any one of the first to eleventh embodiments, wherein the first components further comprise water and a water-immiscible organic solvent.

In a thirteenth embodiment, the present disclosure provides the method of the twelfth embodiment, wherein the water-immiscible organic solvent is a halogenated solvent.

In a fourteenth embodiment, the present disclosure provides the method of the twelfth or thirteenth embodiment, wherein the water-immiscible organic solvent comprises at least one of carbon tetrachloride, hexachloroethane, 1,1,2-trichloro-1,2,2-trifluoroethane, or a hydrofluoroether.

In a fifteenth embodiment, the present disclosure provides the method of any one of the twelfth to fourteenth embodiments, wherein the first components comprise an organic co-solvent.

In a sixteenth embodiment, the present disclosure provides the method of the fifteenth embodiment, wherein the organic co-solvent comprises at least one of acetonitrile, propionitrile, or adiponitrile.

In a seventeenth embodiment, the present disclosure provides the method of any one of the first to sixteenth embodiments, wherein the first components are allowed to react at a temperature of less than 50 °C.

In an eighteenth embodiment, the present disclosure provides the method of the seventeenth embodiment, wherein the first components are not heated above room temperature.

In a nineteenth embodiment, the present disclosure provides the method of any one of the first to eighteenth embodiments, further comprising converting the polyfluorinated dicarboxylic acid to a polyfluorinated dicarboxylic acid halide.

In a twentieth embodiment, the present disclosure provides the method of the nineteenth embodiment, further comprising combining second components comprising the polyfluorinated dicarboxylic acid halide and a metal iodide to provide a polyfluorinated diiodide as the polyfunctional polyfluorinated compound.

In a twenty-first embodiment, the present disclosure provides the method of the nineteenth embodiment, wherein the polyfluorinated dicarboxylic acid halide is a polyfluorinated dicarboxylic acid fluoride, the method further comprising combining third components comprising the polyfluorinated dicarboxylic acid fluoride and hexafluoropropylene oxide to provide a polyfluorinated divinyl ether as the polyfunctional polyfluorinated compound.

In a twenty-second embodiment, the present disclosure provides the method of the nineteenth embodiment, wherein the polyfluorinated dicarboxylic acid halide is a polyfluorinated dicarboxylic acid fluoride, the method further comprising combining fourth components comprising the polyfluorinated dicarboxylic acid fluoride, fluoride ion, and CF₂=CF-CF₂-X, wherein X is Cl, Br, chlorosulfate, fluorosulfate, or trifluoromethyl sulfate, to provide a polyfluorinated diallyl ether as the polyfunctional polyfluorinated compound.

In a twenty-third embodiment, the present disclosure provides a method of the nineteenth embodiment, wherein the polyfunctional polyfluorinated compound comprises at least one of a polyfluorinated diiodide, a polyfluorinated divinyl ether, a polyfluorinated diallyl ether, a polyfluorinated diolefin, or a polyfluorinated dinitrile.

In a twenty-fourth embodiment, the present disclosure provides the method of the twenty-third embodiment, wherein the polyfunctional polyfluorinated compound comprises at least one of a perfluorinated diiodide, a perfluorinated divinyl ether, a perfluorinated diallyl ether, a perfluorinated diolefin, or a perfluorinated dinitrile.

In a twenty-fifth embodiment, the present disclosure provides the method of the nineteenth embodiment, wherein the polyfunctional polyfluorinated compound has at least two different functional groups selected from the group consisting of iodo, vinyl ether, fluorinated allyl ether, olefin, and cyano.

### EXAMPLES

The following specific, but non-limiting, examples will serve to illustrate the present disclosure.

All materials are commercially available, for example from Sigma-Aldrich Chemical Company, Milwaukee, WI, USA, or known to those skilled in the art, unless otherwise stated or apparent.

The following abbreviations are used in this section: mL=milliliters, g=grams, kg=kilograms, mol%= percent by moles, mmol=millimoles, h=hours, NMR=nuclear magnetic resonance, r.t.=room temperature, mmHg=milllimeters of mercury, eq=equivalent. Abbreviations for materials used in this section, as well as descriptions of the materials, are provided in Table 1.

### Materials

**Table 1**

| **Material** | **Details** |
|---|---|
| *c*-C₅F₈ | Perfluorocyclopentene, available from abcr GmbH, Germany |
| RuCl₃×H₂O | Ruthenium(III) chloride hydrate, available from Sigma-Aldrich |
| RuO₂×H₂O | Ruthenium(IV) oxide hydrate, available from Sigma-Aldrich |
| RuO₂ | Ruthenium(IV) oxide, available from Sigma-Aldrich |
| NaIO₄ | Available from Sigma-Aldrich |
| NaOCl (13%) | Carl Roth GmbH + Co. KG |
| NaOH | Available from Sigma-Aldrich |
| ALIQUAT 336 | Water insoluble quaternary ammonium salt made by the methylation of mixed tri octyl/decyl amine, available under the trade designation "ALIQUAT 336" from BASF, Ludwigshafen, Germany. |
| H₅IO₆ | Available from Sigma-Aldrich |
| KBrO₃ | Available from Sigma-Aldrich |
| Water | Deionized water |
| CCl₄ | Available from Sigma-Aldrich |
| CH₃CN | Available from Sigma-Aldrich |
| CH₂Cl₂ | Available from Sigma-Aldrich |
| C₆H₁₂ | Available from Sigma-Aldrich |
| H₂SO₄ | Available from Sigma-Aldrich |
| MeOH | Methanol, available from Sigma-Aldrich |
| EtOAc | Ethyl acetate, availale from Sigma-Aldrich |
| Diethyl ether | Carl Roth GmbH + Co. KG |
| HCl | Available from Sigma-Aldrich |
| CaCl₂ | Available from Sigma-Aldrich |
| FeCl₃ | Available from Sigma-Aldrich |
| Benzotrichloride | α,α,α-Trichlorotoluene, available from Sigma-Aldrich |
| KI | Available from Sigma-Aldrich |
| Na₂SO₃ solution | Saturated solution of Na₂SO₃, available from Sigma-Aldrich, in water |
| MgSO₄ | Available from Sigma-Aldrich |
| F-113 | 1,1,2-Trichloro-1,2,2-trifluoroethane; FlukaAG, Buchs SG, Switzerland |

### Examples 1 through 9 (EX-1 through EX-9)

For EX-1, to a 500 mL three-necked flask equipped with additional funnel and condenser, RuCl₃×H₂O (129 mg, 0.6 mmol, 1.0 mol%), CCl₄ (60 mL) and CH₃CN (30 mL) were transferred. The dark suspension was cooled to 0 °C by an icebath and ice-cooled *c*-C₅F₈ (13.0 g, 61 mmol) was quickly added. To the suspension, H₅IO₆ (74.6 g, 0.33 mol) dissolved in water (310 mL) was added dropwise with intensive stirring at 5 to 6 °C. An exotherm was observed. Afterwards, the cooling bath was removed and the reaction mixture was stirred at r.t. for 12 h. Then, MeOH (1.2 mL) was added. RuO₂ precipitated and was filtered off. The water phase was separated and acidified with H₂SO₄ (10 mL) to pH = 1 (here, the temperature of the water phase was kept at 8 °C or below). The solution was extracted with ether (4 × 150 mL), dried with CaCl₂ and ether was removed in vacuo. After evaporation of the solvent, the residue was dried for 2 h in vacuo (1 mmHg) at 60 °C to form a crystalline solid. The desired diacid was obtained in a yield of 90% (13.1 g, 55 mmol) with a purity of 99.9 mol%.

For EX-2 through EX-11, the same procedure was followed as described for EX-1, but with catalyst, catalyst amount, oxidant, oxidant amount, solvent, time, temperature, and yield of HO₂C-(CF₂)₃-CO₂H as indicated in Table 2.

### Illustrative Examples 1 through 3 (IE-1 through IE-3)

For IE-1 through CE-3, the same procedure was followed as descried for EX-1, but with catalyst, catalyst amount, oxidant, oxidant amount, solvent, time, temperature, and yield of HO₂C-(CF₂)₃-CO₂H as indicated in Table 2.

### Illustrative Example 4

To a solution of NaOCl (13%, 45 mL, 54.3 g, 95 mmol), NaOH (1.9 g, 47.5 mmol), and Aliquat 336 (0.3 g, 0.7 mmol), CH₃CN (7 mL) was added. The reaction mixture was cooled to 10 °C and *c*-C₅F₈ (3.1 g, 14.6 mmol) was added. The flask was sealed and was slowly warmed to r.t. during 16 h with intensive stirring. Afterwards, water (20 mL) was added to the suspension until a full homogenization was observed. The obtained solution was evaporated at r.t. to remove CH₃CN. Next, the reaction mixture was acidified with conc. HCl to pH = 1. The obtained solution was extracted with CH₂Cl₂ (3 × 60 ml) and EtOAc (3 × 50 mL). The EtOAc phase was dried over molecular sieves (4 Ǻ), evaporated and a yellowish residue was distilled in vacuo. The fraction with b.p. 102 - 104 °C at 1 mmHg was collected. The target product was obtained in 32% yield (1.1 g, 4.6 mmol; m.p. 84 - 86 °C; m.p. is in accordance with J. Am. Chem. Soc. 1945, 67, 1235-1237).

**Table 2. Synthesis Details for EX-1 through EX-9 and CE-1 through CE-5**

| Example or Counter Example | Reagent [mol%] | co-Oxidant [eq] | Solvent | Time [h] | T [°C] | Yield of 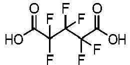 %)^{a} | Other products (mol %)^{b} |
|---|---|---|---|---|---|---|---|
| EX-1 | RuCl₃×H₂O [1.0] | 19% H₅IO₆ [5.25] | H₂O/CCl₄/ CH₃CN | 12 | 23 | 90 | 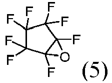 |
| EX-2 | RuCl₃×H₂O [0.5] | 10% NaIO₄ [4.0] | H₂O/CCl₄ | 12 | 24 | 52 | 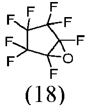 |
| EX-3 | RuO₂×H₂O [0.5] | 10% NaIO₄ [4.2] Aliquat 336 [0.02] | H₂O/CCl₄ | 36 | 24 | 64 | 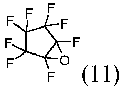 |
| EX-4 | RuO₂×H₂O [0.4] | 10% NaIO₄ [4.0] | H₂O/CCl₄ | 48 | 24 | 72 | 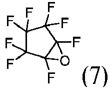 |
| Reference EX-5 | RuCl₃×H₂O [0.5] | 12% NaOCl [4.0] | H₂O/CCl₄ | 36 | 23 | 46 | 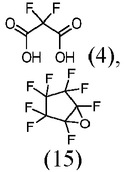 |
| Reference EX-6 | RuCl₃×H₂O [0.8] | 12% NaOCl [5.0] Aliquat 336 [0.03] | H₂O/CCl₄ | 48 | 24 | 44 | 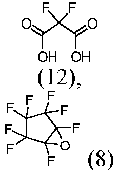 |
| Reference EX-7 | RuO₂×H₂O [0.6] | 12% NaOCl [4.4] | H₂O/CCl₄/ CH₃CN | 14 | 24 | 50 | 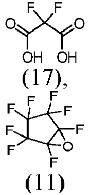 |
| EX-8 | RuO₂×H₂O [0.8] | 19% H₅IO₆ [5.5] | H₂O/CCl₄ | 48 | 24 | 74 | 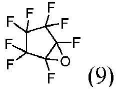 |
| EX-9 | RuO₂×H₂O [0.6] | 19% H₅IO₆ [5.25] | H₂O/C₆H₁₂ / CH₃CN | 24 | 24 | 85 | 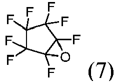 |
| CE-1 | RuO₂×H₂O [0.8] | 6.5% KBrO₃ [6.0] | H₂O/CCl₄ | 24 | 23 | - | - |
| CE-2 | RuO₂×H₂O [0.5] | 6.5% KBrO₃ [6.2] | H₂O/CCl₄/ CH₃CN | 12 | 45 | - | 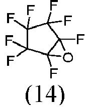 |
| Reference Ex 10 | RuO₂ [0.5] | 12% NaOCl [5.0] | F-113 (CF₂Cl-CFCl₂) | 6 | 5-24 | 6 mol% | 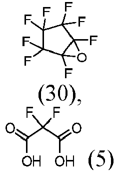 |
| Reference Ex- 11 | RuO₂ [0.1] | 12% NaOCl [5.0] | CCl₄ | | 24 | 8 mol% | 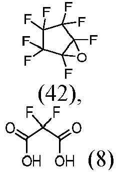 |
| CE-3 | RuO₂ [0.5] | 10% NaIO₄ [5.0] | CCl₄ | 24 | 24 | - | 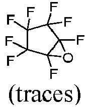 |

| | | | | | | | |
|---|---|---|---|---|---|---|---|
| ^{a)} Isolated yield. ^{b)} According to ¹⁹F NMR ^{c)} Only starting material was found according to ¹⁹F NMR | | | | | | | |

### Example 12 (Ex-12): Synthesis of 2,2,3,3,4,4-hexafluoropentanedioyl dichloride

Anhydrous perfluoroglutaric acid (20.2 g, 84.1 mmol), made as described above, and anhydrous FeCl₃ (210 mg, 1.3 mmol) were placed in a 50 mL three-necked flask equipped with a thermometer and reflux condenser in an inert atmosphere. Then, benzotrichloride (32.5 g, 166.2 mmol) was added. Upon intense stirring, the reaction mixture was slowly warmed to 40 °C during 2 h, and foam formation was observed. The reaction mixture was stirred 4 h at 130 °C and overnight at 24 °C.

The reflux condenser was substituted by a distillation set with a short Vigreux column and the reaction mixture was distilled. The product 2,2,3,3,4,4-hexafluoropentanedioyl dichloride was obtained in 84% yield (19.5 g, 70.4 mmol, b.p. 112-116 °C/760 mmHg).

### Example 13 (EX-13): Synthesis of 1,1,2,2,3,3-hexafluoro-1,3-diiodopropane

2,2,3,3,4,4-hexafluoropentanedioyl dichloride (19.4 g, 70.0 mmol, prepared as described in Ex. 10 and pre-dried KI (31.0 g, 186.7 mol) were placed under argon in a 80 mL Hoke cylinder equipped with magnetic stir bar. The cylinder was stirred 6 h at 203 °C (CO pressure inside was 64 atm). After cooling to r.t., CO was carefully evolved. Diethyl ether (4 × 35 ml) and cold water (4 × 30 ml) were added. The organic phase was separated, washed with a sat. Na₂SO₃ solution (80 mL), and was dried over MgSO₄. After evaporation of solvent, the crude product was distilled with a short Vigreux column. The product 1,1,2,2,3,3-hexafluoro-1,3-diiodopropane was obtained in 70% yield (19.8 g, 49.0 mmol, b.p. 64 - 68 °C/85-92 mmHg).

Various modifications and alterations of this disclosure may be made by those skilled in the art without departing from the scope and spirit of the disclosure, and it should be understood that this invention is only limited by the appended claims.

## Claims

1. A method of making a polyfunctional polyfluorinated compound, the method comprising:
combining first components comprising:
a polyfluorinated cyclic compound having 4 to 7 ring carbon atoms, wherein two of the ring carbon atoms form a double bond or an epoxide; and
ruthenium tetroxide, and
forming a polyfluorinated dicarboxylic acid
wherein the first components comprise a precursor of ruthenium tetroxide wherein the precursor reacts with an oxidant to form ruthenium tetroxide, and wherein the precursor of ruthenium tetroxide comprises ruthenium(IV) oxide hydrate, ruthenium(III) chloride hydrate, or a combination thereof and wherein the oxidant is a periodic acid or salt thereof.

2. The method of claim 1, the polyfluorinated cyclic compound having 4 to 7 ring carbon atoms is substituted by one or more polyfluoroalkyl groups.

3. The method of claim 1 or 2, wherein the polyfluorinated cyclic compound having 4 to 7 ring carbon atoms is a polyfluorinated cyclic olefin.

4. The method of any one of claims 1 to 3, wherein the polyfluorinated cyclic olefin is perfluorocyclopentene.

5. The method of claim 4, further comprising obtaining perfluorocyclopentene from a waste stream.

6. The method of claim 5, wherein the oxidant comprises at least one of a metaperiodate salt, or orthoperiodic acid or a salt thereof

7. The method of any one of claims 1 to 6, wherein the oxidant does not include an oxygen-containing gas.

8. The method of any one of claims 1 to 7, wherein the first components further comprise water and a water-immiscible organic solvent.

9. The method of any one of claims 1 to 8, wherein the first components are allowed to react at a temperature of less than 50 °C.

10. The method of any one of claims 1 to 9, further comprising converting the polyfluorinated dicarboxylic acid to a polyfluorinated dicarboxylic acid halide.

11. The method of claim 10, further comprising combining second components comprising the polyfluorinated dicarboxylic acid halide and a metal iodide to provide a polyfluorinated diiodide as the polyfunctional polyfluorinated compound.

12. The method of claim 10, wherein the polyfluorinated dicarboxylic acid halide is a polyfluorinated dicarboxylic acid fluoride, the method further comprising combining third components comprising the polyfluorinated dicarboxylic acid fluoride and hexafluoropropylene oxide to provide a polyfluorinated divinyl ether as the polyfunctional polyfluorinated compound.

13. The method of claim 10, wherein the polyfluorinated dicarboxylic acid halide is a polyfluorinated dicarboxylic acid fluoride, the method further comprising combining fourth components comprising the polyfluorinated dicarboxylic acid fluoride, fluoride ion, and CF₂=CF-CF₂-X, wherein X is Cl, Br, chlorosulfate, fluorosulfate, or trifluoromethyl sulfate, to provide a polyfluorinated diallyl ether as the polyfunctional polyfluorinated compound.

14. The method of claim 10, wherein the polyfunctional polyfluorinated compound has at least two different functional groups selected from the group consisting of iodo, vinyl ether, fluorinated allyl ether, olefin, and cyano.

## Patentansprüche

1. Ein Verfahren zum Herstellen einer polyfunktionellen polyfluorierten Verbindung, wobei das Verfahren umfasst:
Kombinieren erster Komponenten, umfassend:
eine polyfluorierte cyclische Verbindung mit 4 bis 7 Ringkohlenstoffatomen, wobei zwei der Ringkohlenstoffatome eine Doppelbindung oder ein Epoxid bilden; und
Rutheniumtetroxid, und
Bilden einer polyfluorierten Dicarbonsäure
wobei die ersten Komponenten einen Vorläufer von Rutheniumtetroxid umfassen, wobei der Vorläufer mit einem Oxidationsmittel reagiert, um Rutheniumtetroxid zu bilden, und wobei der Vorläufer von Rutheniumtetroxid Ruthenium(IV)-oxidhydrat, Ruthenium(III)-chloridhydrat oder eine Kombination davon umfasst, und wobei das Oxidationsmittel eine Periodsäure oder ein Salz davon ist.

2. Das Verfahren nach Anspruch 1, wobei die polyfluorierte cyclische Verbindung mit 4 bis 7 Ringkohlenstoffatomen durch eine oder mehrere Polyfluoralkylgruppen substituiert ist.

3. Das Verfahren nach Anspruch 1 oder 2, wobei die polyfluorierte cyclische Verbindung mit 4 bis 7 Ringkohlenstoffatomen ein polyfluoriertes cyclisches Olefin ist.

4. Das Verfahren nach einem der Ansprüche 1 bis 3, wobei das polyfluorierte cyclische Olefin Perfluorcyclopenten ist.

5. Das Verfahren nach Anspruch 4, ferner umfassend das Erhalten von Perfluorcyclopenten aus einem Abfallstrom.

6. Das Verfahren nach Anspruch 5, wobei das Oxidationsmittel mindestens eines von Metaperiodatsalz oder Orthoperiodsäure oder einem Salz davon umfasst.

7. Das Verfahren nach einem der Ansprüche 1 bis 6, wobei das Oxidationsmittel kein sauerstoffhaltiges Gas enthält.

8. Das Verfahren nach einem der Ansprüche 1 bis 7, wobei die ersten Komponenten ferner Wasser und ein nicht wassermischbares organisches Lösungsmittel umfassen.

9. Das Verfahren nach einem der Ansprüche 1 bis 8, wobei man die ersten Komponenten bei einer Temperatur von weniger als 50 °C reagieren lässt.

10. Das Verfahren nach einem der Ansprüche 1 bis 9, ferner umfassend das Umwandeln der polyfluorierten Dicarbonsäure in ein polyfluoriertes Dicarbonsäurehalogenid.

11. Das Verfahren nach Anspruch 10, ferner umfassend das Kombinieren zweiter Komponenten, die das polyfluorierte Dicarbonsäurehalogenid und ein Metalliodid umfassen, um ein polyfluoriertes Diiodid als polyfunktionelle polyfluorierte Verbindung bereitzustellen.

12. Das Verfahren nach Anspruch 10, wobei das polyfluorierte Dicarbonsäurehalogenid ein polyfluoriertes Dicarbonsäurefluorid ist, wobei das Verfahren ferner das Kombinieren dritter Komponenten umfasst, die das polyfluorierte Dicarbonsäurefluorid und Hexafluorpropylenoxid umfassen, um einen polyfluorierten Divinylether als polyfunktionelle polyfluorierte Verbindung bereitzustellen.

13. Das Verfahren nach Anspruch 10, wobei das polyfluorierte Dicarbonsäurehalogenid ein polyfluoriertes Dicarbonsäurefluorid ist, wobei das Verfahren ferner das Kombinieren vierter Komponenten umfasst, die das polyfluorierte Dicarbonsäurefluorid, Fluoridion und CF₂=CF-CF₂- X umfassen, wobei X Cl, Br, Chlorsulfat, Fluorsulfat oder Trifluormethylsulfat ist, um einen polyfluorierten Diallylether als polyfunktionelle polyfluorierte Verbindung bereitzustellen.

14. Das Verfahren nach Anspruch 10, wobei die polyfunktionelle polyfluorierte Verbindung mindestens zwei unterschiedliche funktionelle Gruppen aufweist, ausgewählt aus der Gruppe bestehend aus lod, Vinylether, fluoriertem Allylether, Olefin und Cyano.

## Revendications

1. Procédé de fabrication d'un composé polyfluoré polyfonctionnel, le procédé comprenant : la combinaison de premiers composants comprenant :
un composé cyclique polyfluoré ayant 4 à 7 atomes de carbone de cycle, dans lequel deux des atomes de carbone de cycle forment une double liaison ou un époxyde ; et
du tétroxyde de ruthénium, et
la formation d'un acide dicarboxylique polyfluoré
dans lequel les premiers composants comprennent un précurseur de tétroxyde de ruthénium dans lequel le précurseur réagit avec un oxydant pour former du tétroxyde de ruthénium, et dans lequel le précurseur de tétroxyde de ruthénium comprend de l'oxyde de ruthénium (IV) hydraté, du chlorure de ruthénium (III) hydraté, ou une combinaison de ceux-ci et dans lequel l'oxydant est un acide périodique ou un sel de celui-ci.

2. Procédé selon la revendication 1, le composé cyclique polyfluoré ayant 4 à 7 atomes de carbone de cycle est substitué par un ou plusieurs groupes polyfluoroalkyle.

3. Procédé selon la revendication 1 ou 2, dans lequel le composé cyclique polyfluoré ayant 4 à 7 atomes de carbone de cycle est une oléfine cyclique polyfluorée.

4. Procédé selon l'une quelconque des revendications 1 à 3, dans lequel l'oléfine cyclique polyfluorée est du perfluorocyclopentène.

5. Procédé selon la revendication 4, comprenant en outre l'obtention de perfluorocyclopentène à partir d'un courant d'effluents.

6. Procédé selon la revendication 5, dans lequel l'oxydant comprend au moins un parmi un sel métaperiodate, ou un acide orthoperiodique ou un sel de celui-ci.

7. Procédé selon l'une quelconque des revendications 1 à 6, dans lequel l'oxydant n'inclut pas de gaz contenant de l'oxygène.

8. Procédé selon l'une quelconque des revendications 1 à 7, dans lequel les premiers composants comprennent en outre de l'eau et un solvant organique non miscible dans l'eau.

9. Procédé selon l'une quelconque des revendications 1 à 8, dans lequel on laisse les premiers composants réagir à une température inférieure à 50 °C.

10. Procédé selon l'une quelconque des revendications 1 à 9, comprenant en outre la conversion de l'acide dicarboxylique polyfluoré en un halogénure d'acide dicarboxylique polyfluoré.

11. Procédé selon la revendication 10, comprenant en outre la combinaison de deuxièmes composants comprenant l'halogénure d'acide dicarboxylique polyfluoré et un iodure de métal pour fournir un diiodure polyfluoré en guise de composé polyfluoré polyfonctionnel.

12. Procédé selon la revendication 10, dans lequel l'halogénure d'acide dicarboxylique polyfluoré est un fluorure d'acide dicarboxylique polyfluoré, le procédé comprenant en outre la combinaison de troisièmes composants comprenant le fluorure d'acide dicarboxylique polyfluoré et de l'oxyde d'hexafluoropropylène pour fournir un éther divinylique polyfluoré en guise de composé polyfluoré polyfonctionnel.

13. Procédé selon la revendication 10, dans lequel l'halogénure d'acide dicarboxylique polyfluoré est un fluorure d'acide dicarboxylique polyfluoré, le procédé comprenant en outre la combinaison de quatrièmes composants comprenant le fluorure d'acide dicarboxylique polyfluoré, un ion fluorure, et CF₂=CF-CF₂- X, dans lequel X est Cl, Br, chlorosulfate, fluorosulfate, ou trifluorométhyl-sulfate, pour fournir un éther diallylique polyfluoré en guise de composé polyfluoré polyfonctionnel.

14. Procédé selon la revendication 10, dans lequel le composé polyfluoré polyfonctionnel a au moins deux groupes fonctionnels différents choisis dans le groupe constitué d'iodo, éther vinylique, éther allylique fluoré, oléfine, et cyano.
